# EUROPEAN PATENT SPECIFICATION

(11) **EP 2 655 390 B1**
(45) Date of publication and mention of the grant of the patent: **27.03.2019**
(21) Application number: 11851169.0
(22) Date of filing: 21.12.2011
(51) Int. Cl.: C07J 63/00, C07H 1/08, C07H 13/08, A61K 36/73

(54) **PURIFIED CARDIOGENIN ISOMER AND RELATED METHODS**
GEREINIGTES CARDIOGENIN-ISOMER UND ZUGEHÖRIGE VERFAHREN
ISOMÈRE DE CARDIOGÉNINE PURIFIÉ ET PROCÉDÉS APPARENTÉS

(30) Priority: 23.12.2010 US 201061426929 P
(43) Date of publication of application: 30.10.2013
(73) Proprietor: Huya Bioscience International LLC, San Diego, CA 92130 (US)
(72) Inventor: KYAS, Andreas, CH-5001 Aarau (CH); FREUND, Ernst, CH-5001 Aarau (CH); SCHLÖRKE, Oliver, CH-5001 Aarau (CH); PATERNITI, James, R., San Diego, CA 92130 (US); ELLIOTT, Gary, San Diego, CA 92130 (US); LILL, Jorg, CH-5001 Aarau (CH); ROGALL, Lars, CH-5001 Aarau (CH); MENIA, Dario, CH-5001 Aarau (CH)
(74) Representative: Schiweck Weinzierl Koch Patentanwälte Partnerschaft mbB
(86) International application number: PCT/US2011/066469
(87) International publication number: WO 2012/088264

(56) References cited:
- WO-A1-2007/049089
- WO-A1-2010/143062
- CA-A1- 2 209 222
- US-A- 5 916 919
- US-A1- 2003 118 635
- US-A1- 2009 028 959
- FA-QUAN ZENG ET AL: "The anticoagulant effects of Geum japonicum extract and its constituents", PHYTOTHERAPY RESEARCH, JOHN WILEY & SONS LTD. CHICHESTER, GB, vol. 12, no. 2, 1 March 1998 (1998-03-01), pages 146-148, XP008150177, ISSN: 0951-418X, DOI: 10.1002/(SICI)1099-1573(199803)12:2<146::A ID-PTR204>3.0.CO;2-5 [retrieved on 1998-12-18]
- SHIGENAGA S ET AL: "Triterpenoids and glycosides from Geum japonicum", PHYTOCHEMISTRY, PERGAMON PRESS, GB, vol. 24, no. 1, 1 January 1985 (1985-01-01), pages 115-118, XP026620680, ISSN: 0031-9422, DOI: 10.1016/S0031-9422(00)80818-3 [retrieved on 1985-01-01]
- ZHANG ET AL: "Cellulose tris(3,5-dichlorophenylcarbamate) immobilised on silica: A novel chiral stationary phase for resolution of enantiomers", JOURNAL OF PHARMACEUTICAL AND BIOMEDICAL ANALYSIS, NEW YORK, NY, US, vol. 46, no. 5, 21 March 2008 (2008-03-21) , pages 882-891, XP022552158, ISSN: 0731-7085
- PIRZADA Z ET AL: "Systematic evaluation of new chiral stationary phases for supercritical fluid chromatography using a standard racemate library", JOURNAL OF CHROMATOGRAPHY, ELSEVIER SCIENCE PUBLISHERS B.V, NL, vol. 1217, no. 7, 12 February 2010 (2010-02-12), pages 1134-1138, XP026857308, ISSN: 0021-9673 [retrieved on 2009-10-08]
- CHENG, L. ET AL.: 'A Plant-Derived Remedy for Repair of Infarcted Heart' PLOS ONE vol. 4, no. 2, February 2009, page E4461, XP055112799

## Description

### CROSS-REFERENCE TO RELATED PATENT APPLICATIONS

This application claims priority to United States provisional application number 61/426,929, filed December 23, 2010.

### BACKGROUND OF THE INVENTION

A methanolic extract of *Geum japonicum,* denoted "EGJ," has been shown to have activity in promoting regeneration of myocardium. Cheng et al., PLoS One 4(2): e4461 (2009). That activity was attributed to an EGJ component, a cardiac glycoside called "cardiogenin" (C₃₆H₅₈O₁₁), which is (2α, 3β, 4α)-2,3,19,23-tetrahydroxy-urs-12-en-28-oic acid β-D-glucopyranosyl ester. The chemical structure ascribed to cardiogenin possesses 16 chiral centers, giving rise to the theoretical possibility of many stereoisomers.

In this context, Cheng *et al.* described a procedure in a manner that suggests the obtention of single stereoisomer of cardiogenin. There was insufficient detail provided, however, for the practicable isolation of a cardiogenin composition characterized by least 90% purity, and Cheng *et al.* did not themselves describe the purity of "isolated" cardiogenin. Pursuant to the Cheng methodology, therefore, it was unknown whether and to what extent impurities existed in the resultant composition.

### SUMMARY OF THE INVENTION

Against this background of the conventional technology, the present inventors discovered that "cardiogenin" extracted and purified from EGJ, using the method described by Cheng *et al.* (2009), actually comprises two, closely eluting isomers of the same mass. Accordingly, the inventors developed an approach for separating the previously unrecognized cardiogenin major isomer, which was found to be active, from the minor isomer, which is inactive. Via this approach, the inventors succeeded in extracting from EGJ a cardiogenin major-isomer composition that is substantially free of the minor isomer (hereafter, "isolated cardiogenin major isomer").

Thus, the inventive methodology provides an isolated cardiogenin major isomer having at least 98% (a/a) HPLC purity at 210 nm (hereafter, "substantial purity"). Substantial purity would be achieved by crystallizing the isolated cardiogenin major isomer to separate the impurities. In this context, "a/a" denotes the percent area of a peak of interest in a chromatogram to the total area of all other peaks in the chromatogram at a specific wavelength. The a/a value serves here as the unit measure of optical purity for cardiogenin isomer.

Herein described are the major and the minor cardiogenin isomers, the corresponding aglycone thereof, and related compositions, as well as methodology for making and using them. Herein described is isolated cardiogenin major isomer, which can be described, for example, in terms of the formula:

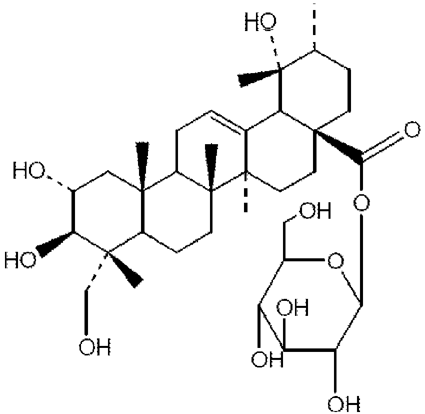

Further described is aglycone of the isolated cardiogenin major isomer with at least 92% purity by HPLC. The aglycone can be described, for example, in terms of the formula:

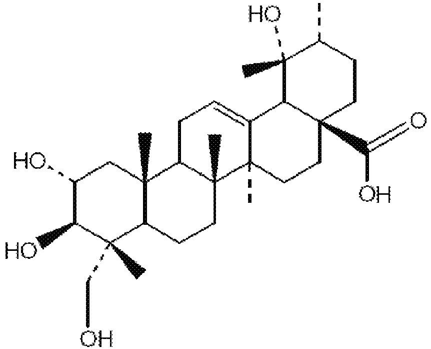

The cardiogenin major isomer preferably is present in substantial purity. An illustrative embodiment of this state is the compound with a HPLC purity of 98.97% (a/a) at 210 nm. Further, a pharmaceutical composition is described that comprises the isolated cardiogenin major isomer and/or its corresponding aglycone, as well as a pharmaceutically acceptable carrier.

The invention also provides a methodology for isolating the major isomer of cardiogenin. The inventive methodology comprises (A) obtaining an extract from the methanol extract of *Geum japonicum* and (B) subjecting the extract to chiral phase chromatography or supercritical fluid chromatography, whereby the major isomer is obtainable in isolated form. An embodiment involving chiral phase chromatography can entail, for instance, the use of a Chiralpak® IC™ column, a product of Chiral Technologies, Inc. (West Chester, PA). The inventive methodology for isolating the cardiogenin major isomer also may comprise crystallizing the composition.

Another aspect the invention relates to an improvement on the chromatographic procedures of Cheng *et al.* (2009), comprising (A) precipitating and filtering an methanolic/water solution of *Geum japonicum* to remove of unwanted solids, (B) phase-separative extracting the methanol/water solution with dichloromethane and tert-butyl methyl ether (TBME), and then (C) extracting with n-butanol. The improved chromatographic procedures of the invention also may comprise subjecting a composition comprised of the major isomer of cardiogenin to low-pressure adsorption chromatography (Diaion HP-20 and silica gel), using an optimized mass ratio of resin to material load (typically, about 15:1) and a step gradient of methanol/water, followed by low pressure silica gel chromatography using an optimized mass ratio of resin to material load (about 20:1) and a step gradient of dichloromethane/methanol. In another embodiment, the improvement over Cheng *et al.* (2009) further comprises subjecting a major isomer-containing composition to high-pressure, reverse-phase chromatography (HPRC), employing aqueous buffer and methanol mobile phases in a gradient program. In this regard, the HPRC can involve using a Luna® C18 (2) column, a product of Phenomenex, Inc. (Torrance, CA).

### BRIEF DESCRIPTION OF FIGURES

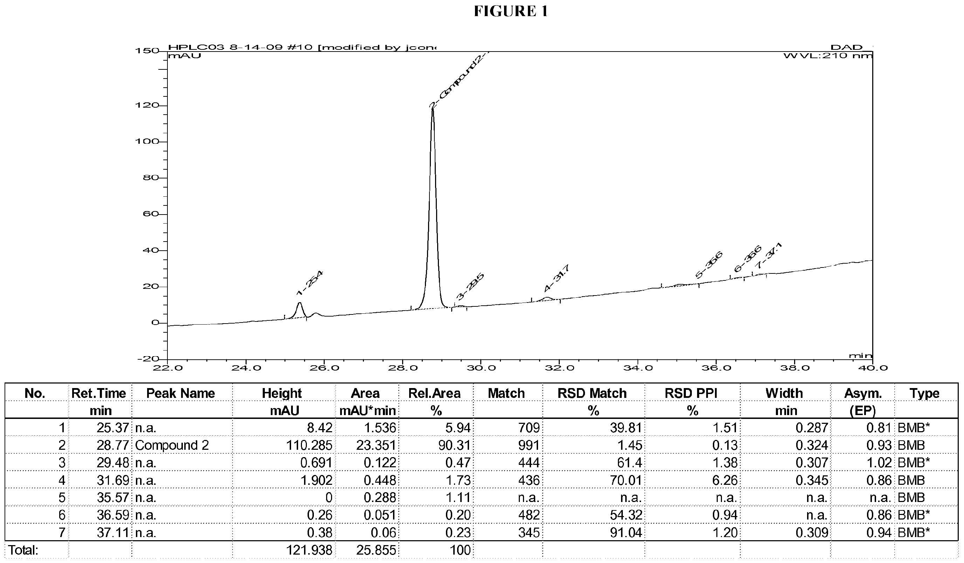

Figure 1 illustrates results from obtaining a semi-purified cardiogenin composition via the method of Cheng *et al.* (2009), in which the cardiogenin diastereoisomers identified by the present inventors are not resolved, one from the other.
Figure 2 depicts data from an HPLC and LC-MS analysis, pursuant to the invention, of semi-purified cardiogenin material produced with the method of Cheng *et al.* (2009). In relation to two peaks, resolved one form the other, the analysis confirms the presence of two cardiogenin isomers. The closely eluting peaks, evident in the HPLC chromatogram, display the same molecular ion acetate and trifluoroacetyl (TFA) adducts, indicating that they are structural isomers.
Figure 3 presents the ¹H-NMR spectra of the semi-purified cardiogenin reference material, mentioned above. The spectra confirm the presence of two cardiogenin isomers.
Figure 4 illustrates results from producing a cardiogenin composition by extraction, further separation by Dianion HP-20 followed by silica gel, and finally reverse phase chromatography. As shown, the composition comprises the two cardiogenin isomers, fully resolved via an optimized-purity HPLC method, with a combined (pre-separation) HPLC purity of 92.6% (a/a) at 210 nm.
Figure 5 shows a schematic overview of methodology for isolating the cardiogenin major isomer, in accordance with the invention.
Figure 6 depicts results from an HPLC purity report, showing the purity of the isolated cardiogenin major isomer after final crystallization to be 98.97% (a/a) at 210 nm.
Figure 7 presents ¹H-NMR spectra that confirm the identity of isolated cardiogenin major isomer, with the removal of minor isomer resonances (see Fig. 3) in the vicinity of 2.3 PPM and 5.375 PPM.
Figure 8 depicts a three-dimensional skeletal model (a) of X-ray crystallographic data for the cardiogenin major isomer. Also depicted is a skeletal formula (b), which is a two-dimensional rendition of (a). Representation (a) shows inter-molecular hydrogen bonds (dash lines) between prescribed oxygen atoms of cardiogenin and hydrogens of water of crystallization.
Figure 9 shows a C18 reverse phase chromatography profile of the cardiogenin major isomer ("HUYA-1"), isolated in accordance with the invention. (How do we describe the 24.254 minor peak?)
Figure 10 shows a C18 reverse phase chromatography profile of the cardiogenin minor isomer ("HUYA-2"), isolated in accordance with the invention. HUYA-1 and HUYA-2 show similar retention time.
Figure 11 shows a C18 reverse phase chromatography profile of the aglycone of the cardiogenin minor isomer ("HUYA-3"), obtained in accordance with the invention.
Figure 12 shows a C18 reverse phase chromatography profile of the aglycone of the cardiogenin major isomer ("HUYA-4"), obtained in accordance with the invention. HUYA-3 and HUYA-4 show similar retention time.
Figure 13 shows a C18 reverse phase chromatography profile of a cardiogenin composition isolated according to the method of Cheng *et al.,* 2009 ("Car"). The retention time of Car is similar to that of HUYA-1 and HUYA-2, respectively.

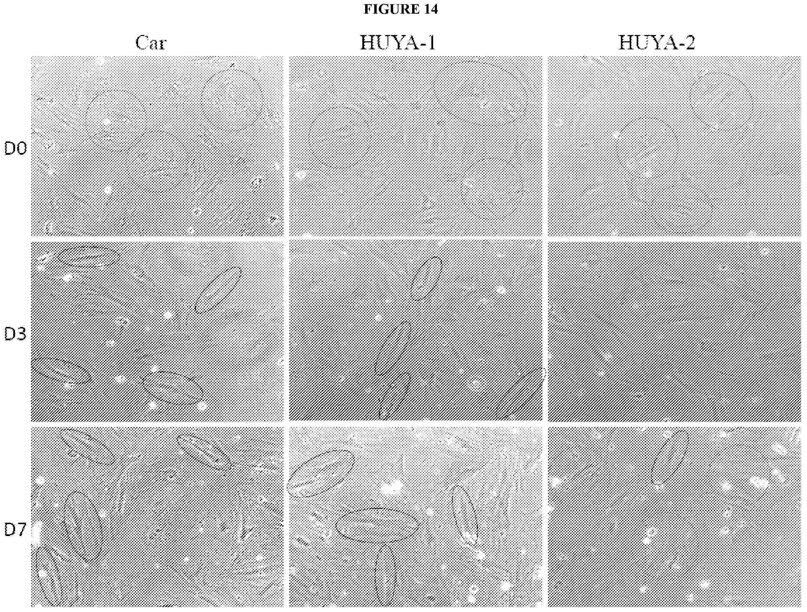

Figure 14 presents photomicrographs that illustrate the activity of Car, HUYA-1 and HUYA-2, each 10 µg/ml, in inducing the cardiogenic morphological transition of mesenchymal stem cells (MSCs). **D0,** cultures of MSCs were set up before any treatment. The morphology of the MSCs was characterized by flat, irregular, low refracted and well-spread shapes (circles). **D3,** sample of the cultured MSCs were treated for 3 days with compounds as respectively labeled. Some of the Car- and HUYA-1-treated MSCs (∼31%) were observed to undergo narrowing and to become more refractive (ovals). By contrast, the HUYA-2-treated MSCs did not show clear morphological changes (circles). **D7,** the cultured MSCs samples were treated for 7 days with compounds as respectively labeled. More MSCs (∼48%) in Car- and HUYA-1-treated cultures underwent narrowing and became more refractive (circles). Again, the HUYA-2-treated MSCs displayed no significant morphological change (circles).

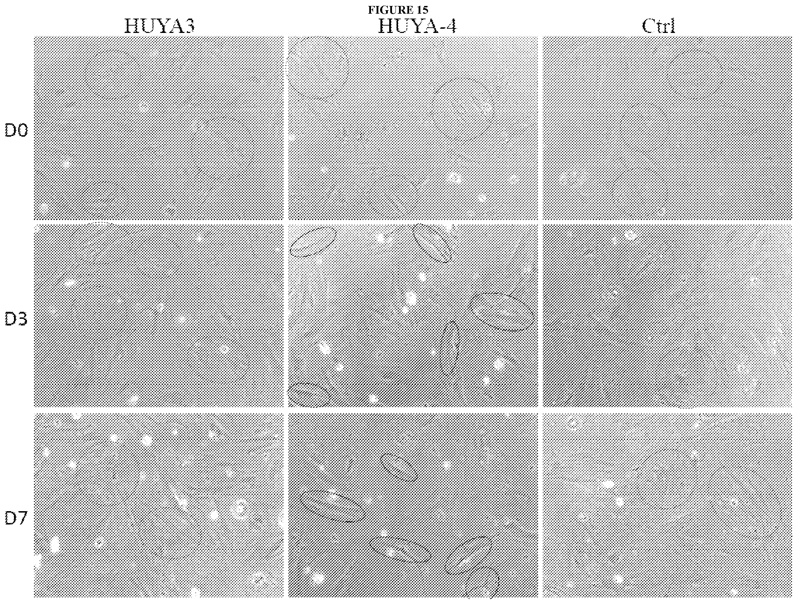

Figure 15 provides a comparison, via photomicrographs, of HUYA-3 (10 µg/ml) with HUYA-4 (10 µg/ml) in inducing cardiogenic morphological transition of MSCs. Ctrl denotes the MSCs treated with vehicle (10% DMSO in equivalent volume). **D0,** the MSCs cultures were set before any treatment. The morphology of the MSCs was characterized by flat, irregular, low refracted and well- spread shapes (circles). **D3,** sample of the cultured MSCs were treated for 3 days with compounds as respectively labeled. Some of the HUYA-4-treated MSCs (∼20%) showed narrowing and a more refractive phenotype (ovals). The HUYA-3- and vehicle-treated MSCs did not show significant morphological changes (circles), however. **D7,** the cultured MSCs samples were treated for 7 days with compounds as respectively labeled. A similar amount of the MSCs (∼22%) in HUYA-4-treated cultures became narrowing and more refractive (ovals). By contrast, the HUYA-3- and vehicle-treated MSCs showed no significant morphological changes (circled).

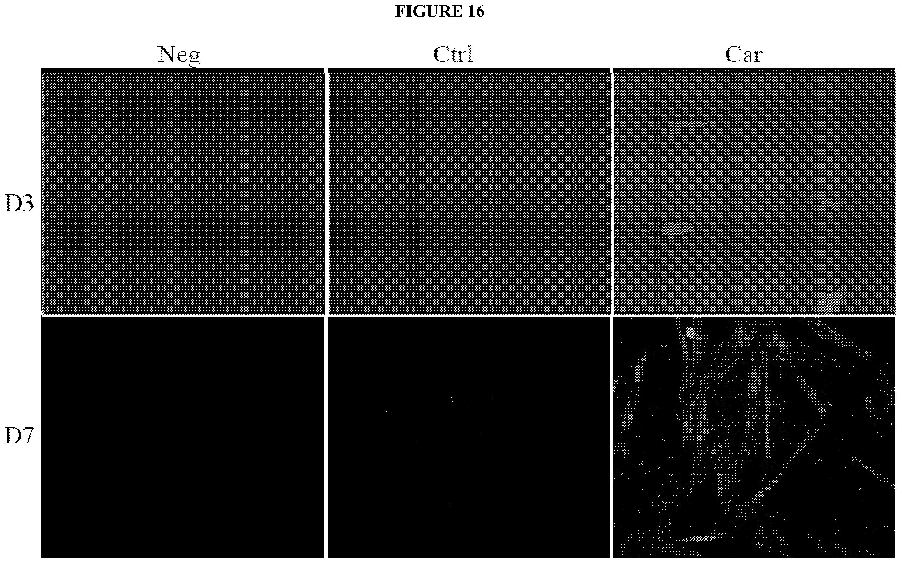

Figure 16 depicts immunofluorescence staining for expression of cardiogenic differentiation markers, Mef2a (fluorescence in **D3**) and beta, MHC beta (fluorescence in **D7**). **D3** and **D7,** the cultured MSCs samples were treated with the compounds, as labeled, for 3 and 7 days, respectively. **Neg** is the negative control of MSCs culture, with no use of the first antibody specific to Mef2a or MHC beta, showing negative signals of Mef2a (**D3**) and MHC (**D7**) staining. **Ctrl** represents the cultured MSCs treated with the equivalent volume of 10% DMSO, with almost no positive Mef2a (**D3**) and MHC beta (**D7**) signals observed. **Car** is the MSCs culture treated with cardiogenin, showing that approximately 13% of the treated cells displayed Mef2a-positive staining, as indicated by the fluorescence (**D3**), and 17% of the treated cells showed MHC-positive signals (fluorescence) when the cells were treated for 7 days (**D7**).

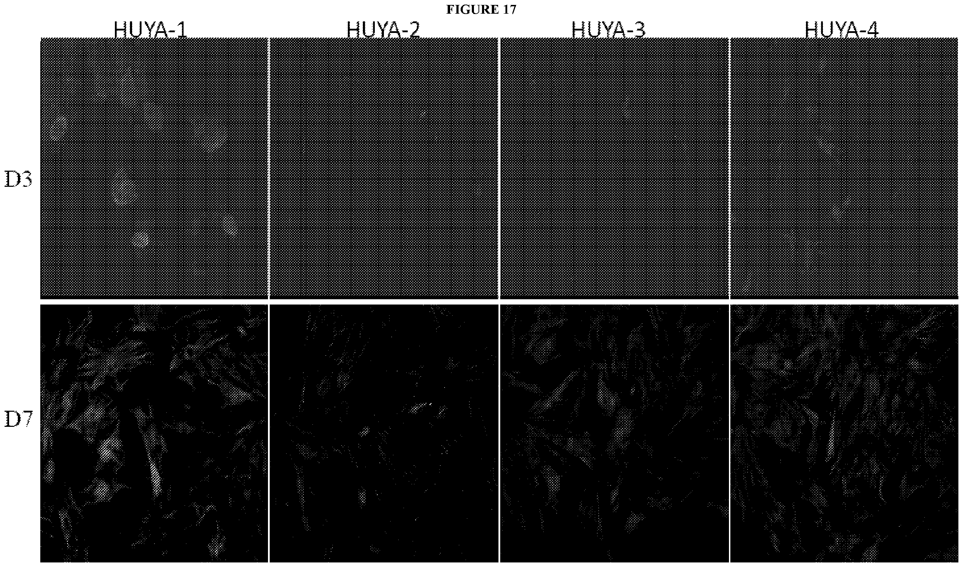

Figure 17 depicts immunofluorescence staining for expressions of early cardiogenic differentiation marker, Mef2a (fluorescence in **D3**) and cardiac specific myosin heavy chain beta, MHC (fluorescence in **D7**). **D3** and **D7,** the cultured MSCs samples were treated with the compounds, as labeled, for 3 and 7 days, respectively. **HUYA-1** represents the MSCs culture that was treated with HUYA-1, showing that approximately 15% of the treated cells displayed Mef2a-positive staining, as indicated by the red signals (**D3**), and that 20% of the treated cells showed MHC-positive signals (fluorescence) when the cells were treated for 7 days (**D7**). **HUYA-2** is the MSCs culture that was treated with HUYA-2, showing almost no positive signals for Mef2a (**D3**) and ∼3% positive signals for MHC beta (**D7**). **HUYA-3** represents the HUYA-3-treated MSCs, with little positive Mef2a (**D3**) and MHC beta (**D7**) signals observed. **HUYA-4** denotes the HUYA-4-treated MSCs culture, showing that approximately ∼6% of the treated cells displayed Mef2a-positive signals (**D3**) and ∼8% of the treated cells showed MHC-beta positive signals (**D7**).

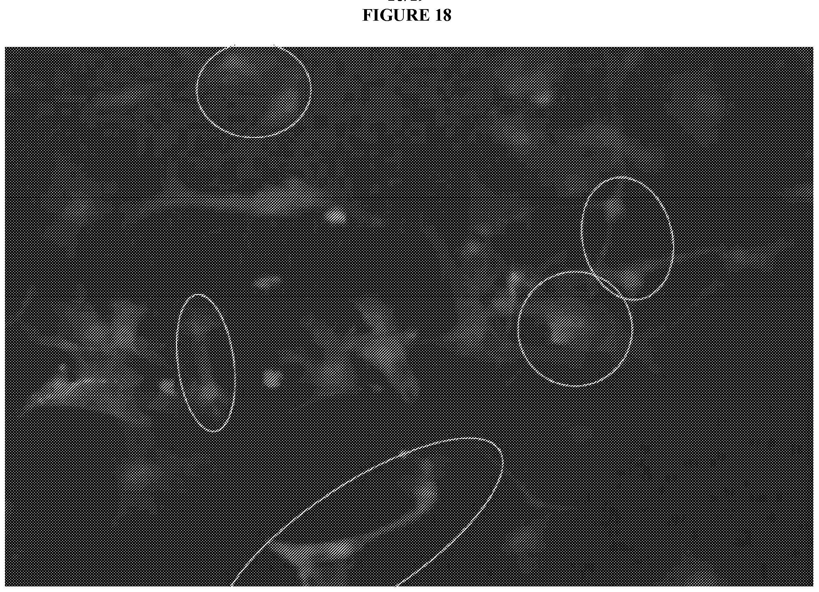

Figure 18 depicts HUYA-1-induced differentiation of GFP-MSCs into beating cardiac myocytes in co-culture system. This screenshot was taken from a video showing beating GFP-positive myocytes (enclosed areas) differentiated from the GFP-MSCs, which were co-cultured with rat cardiac myocytes and fibroblasts and then treated with HUYA-1. Since the culture contained non-GFP myocytes and fibroblasts and GFP-MSCs, any beating cells with GFP-positive signals that were identified must have differentiated from GFP-MSCs. This shows that HUYA-1 can enhance the cardiogenic differentiation of MSCs into beating cardiac myocytes.
Figure 19 presents a method for converting the isolated cardiogenin major isomer to its corresponding aglycone.

### DETAILED DESCRIPTION

Myocardial infarction due to coronary artery disease is one of the leading causes of premature death. One solution is to replace the infarcted heart tissue with regenerated myocardium from endogenous progenitor pools or exogenously introduced stem cells. A methanolic extract of *Geum japonicum* was shown by Cheng *et al.* to have such potential.

As noted above, although Cheng *et al.* described their procedure in a manner suggesting the obtention of a single stereoisomer of cardiogenin, there was insufficient detail for the practicable isolation of a cardiogenin composition characterized by at least 95 - 98% (a/a) HPLC purity at 210 nm, a typical regulatory expectation for modern, small-molecule pharmaceutical substances. Moreover, Cheng *et al.* did not describe the purity of "isolated" cardiogenin. Pursuant to the Cheng methodology, it was unknown whether impurities existed in the resultant composition and, if they did exist, the extent of such impurities.

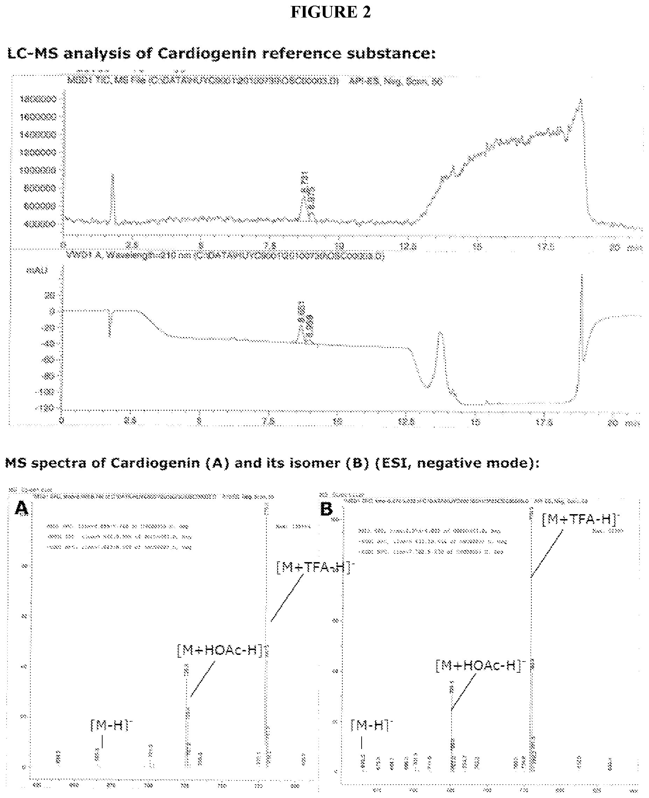

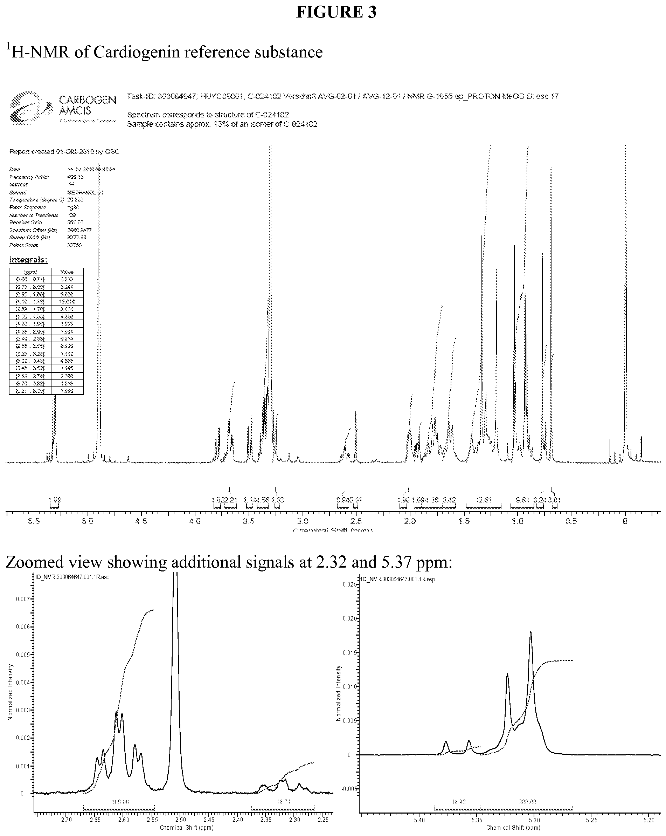

During an evaluation of the conventional method of extracting cardiogenin from EGJ, an HPLC assay method was developed to analyze the purity of the extracted cardiogenin, with an expectation that the extracted composition would comprise a single stereoisomer of cardiogenin. Surprisingly, using a HPLC assay method that was improved over that employed by Cheng *et al.,* the present inventors observed that the conventional method of extracting cardiogenin instead yields a mixture of two closely eluting isomers with identical mass. Subsequently, both LC-MS and ¹H-NMR spectra analyses were performed, each method independently confirmed the discovery that the "cardiogenin" produced via Cheng's method actually comprises two cardiogenin isomers. The LC-MS results are shown in Figure 2, and ¹H-NMR spectra are shown in Figure 3.

The inventors also found that, following the Cheng methodology, the HPLC purity of the isolated cardiogenin major isomer was only about 75.73% (a/a) at 210 nm, with about 16.87% of the HPLC impurities attributable to the minor isomer. Moreover, the isolated cardiogenin major isomer was found to be biologically active, the impurities to be biologically inactive.

### ISOLATING THE MAJOR ISOMER OF CARDIOGENIN

To obtain isolated cardiogenin major isomer with substantial purity, a method was developed to remove impurities, including the previously unrecognized minor isomer of cardiogenin. The extraction procedure taught by Cheng *et al.* (2009) utilizes chloroform, ethyl acetate and finally n-butanol phase separative extraction against water. The n-butanol phase has been retained for further purification and other organic phases were discarded. It has been found that considerable loss of cardiogenin occurs with the ethyl acetate extraction, and the inventors have determined that chloroform cannot be used on an industrial manufacturing scale, given toxicity risks.

Accordingly, an optimization of the extraction process was undertaken. First, the EGJ extraction process was improved by introduction of a methanol re-slurry of EGJ, followed by filtration, concentration and dilution with water and a second Celite-aided filtration. These steps remove from the EGJ extract approximately 50% solids, which are undesired materials, and help to reduce subsequent emulsion formation upon the ensuing organic phase separation. The filter cake is devoid of cardiogenin when the filtration solids are analyzed. Next chloroform extraction of the aqueous/methanol filtrate was replaced with dichloromethane to avoid using highly toxic chloroform, a solvent that presents both an operator safety risk and an environmental risk. An extraction with TBME was added, since this extraction had been shown to remove impurities that elute during HPLC purification at retention times close to that of cardiogenin, without appreciably removing cardiogenin from the methanol/water filtrate. The aqueous methanolic phase was converted io a saturated sodium chloride solution and extracted with n-butanol, which removed cardiogenin from the aqueous/methanol phase with good overall recovery of cardiogenin.

The chromatographic separations taught by Cheng *et al.* (2009) haven been incorporated, including low-pressure Diaion HP-20 adsorption chromatography, followed by low-pressure normal phase silica and finally high pressure reverse phase chromatography, to increase the overall purity of the isomeric mixture. The Diaion and normal-phase silica chromatography are conducted generally as taught by Cheng *et al.,* although conditions are optimized. In particular, Diaion chromatography has been optimized by definition of the optimal mass ratio of resin to material load (15:1) and by the use of a step gradient starting with 20% MEOH/water, increasing in 10% increments to 80% MEOH/water. Silica gel chromatography has been optimized by definition of the optimal mass ratio of resin to material load (20:1), with replacement of chloroform with dichloromethane in the mobile phase, for operator and environmental safety considerations described above, and the use of a stepwise gradient of dichloromethane/methanol ranging from dichloromethane/methanol 90%:10% to dichloromethane/methanol 80%:20%.

The process has been improved further by the introduction of either chiral phase chromatography or supercritical fluid chromatography, after the high pressure reverse phase chromatography, to allow separation of the two closely eluting enantiomers of cardiogenin. Finally the separated major isomer of cardiogenin was crystallized from methanol/water to increase its purity, removing low level impurities seen throughout the elution profile of the HPLC purity method. In accordance with the method, the invention provides the major isomer of cardiogenin in at least 98% HPLC purity with an overall yield from EGJ to cardiogenin exceeding that reported by Cheng *et al.*

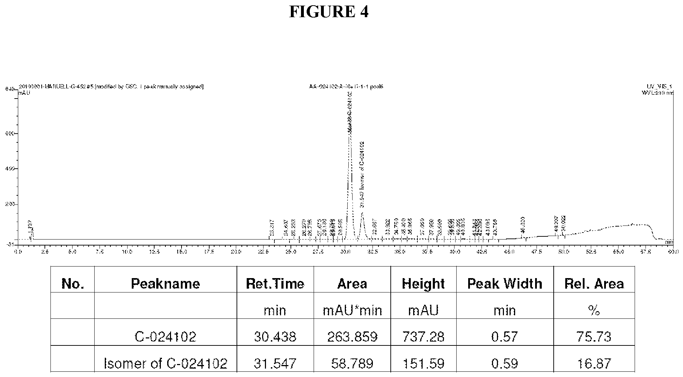

In this regard, the category of suitable reverse phase chromatography techniques encompasses any chromatographic method that uses a non-polar stationary phase. Polar compounds are eluted first while non-polar compounds are retained. The column can be octadecyl carbon chain (C18)-bonded silica. The eluent can be a mixture of ACN and 20 mM NH₄OAc (pH=7). The sample then is dissolved at 47 g/L in MeOH:Buffer = 50:50 (v:v). The temperature for the elution can be room temperature. A mobile phase gradient transitions in a linear fashion from 80% 20 mM ammonium acetate/acetonitrile to 65% 20 mM ammonium acetate/acetonitrile. The flow rate of the column can be 15 mL/min. on an ID=2 cm column. The presence of cardiogenin can be detected by HPLC at 210 nm. As can be calculated from the data shown in Figure 4, the absorptive units peak ratio of the major isomer against the minor isomer is roughly 4.5:1, before separation of the two cardiogenin isomers.

The two cardiogenin isomers are separated by chiral phase chromatography. In this regards, category of chiral phase chromatography techniques encompasses any column chromatography in which the stationary phase contains a single enantiomer of a chiral compound, rather than being achiral. Chiral stationary phase selection is critical to achieve adequate separation. The two isomers of cardiogenin elute from the column at different times because of their transiently different solubility characteristics when bound to the chiral column stationary phase. The column can be a Chiralpak® IC™ column. The eluent can be a mixture of IPA:MTBE=50:50 (v:v). The sample can be dissolved in IPA/MTBA=50:50 (v:v) or neat IPA. The temperature for the elution can be room temperature. The flow rate of the column can be 1 mL/min on an analytical column or higher flow rate on semipreparative columns using an isocratic mobile phase of IPA:MTBE=50:50 (v:v). The presence of cardiogenin can be detected by HPLC at 210 nm.

The two cardiogenin isomers also can be separated by supercritical fluid chromatography, as described, for example, by Anton & Berger, SUPERCRITICAL FLUID CHROMATOGRAPHY WITH PACKED COLUMNS (1st ed. 1997). In this regard, the category of suitable supercritical fluid chromatography techniques encompasses normal stationary phase for separating chiral compounds. Thus, the column can be a Chiralpak® IC™ column, other Chiralpak® stationary phase columns or a C18 column, but preferably is a Chiralpak® IC™ column. The gradient eluent can be CO₂/MeOH or CO₂/ACN. The presence of cardiogenin can be detected by HPLC at 210 nm.

The purity of the isolated cardiogenin major isomer can be increased further by crystallization. In this regard, the "crystallization" category encompasses any method for forming solid crystals of the cardiogenin major isomer, typically by precipitating from a solution, melt, or gas. In a preferred embodiment, the cardiogenin major isomer is dissolved in 10 volumes of methanol at 40°C. Then 40 volumes of water are added slowly, at the same temperature, over a period of about 50 minutes, during which crystallization of the cardiogenin major isomer occurs.

Pursuant to the above-described methodology, cardiogenin major isomer can be isolated from EGJ with a HPLC purity of 98.97% (a/a) at 210 nm and potency by NMR assay of 95.50% (w/w). The isolated cardiogenin major isomer is a stable compound when stored in either MTBE/IPA=50:50 or as solid. It also is resistant to thermal stress at 40°C.

The structure of the cardiogenin major isomer is depicted below:

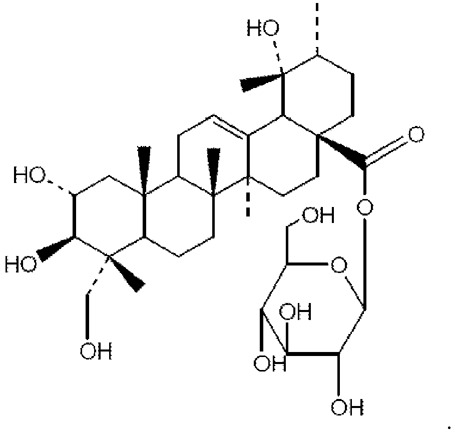

### AGLYCONE OF THE MAJOR ISOMER OF CARDIOGENIN

The corresponding aglycone can be converted from the isolated cardiogenin major isomer, a polycyclic glycoside, by hydrolysis of the ester linkage that connects the sugar moiety to the polycyclic core of the molecule. Such hydrolysis can be accomplished by either of two approaches:
(a) Acid-catalyzed hydrolysis, which involves the use of a dilute aqueous solution of a mineral acid to effect cleavage of the ester. The resultant products are the sugar and the free carboxylic acid form of cardiogenin.
(b) Base-catalyzed hydrolysis (saponification), in which a base such as sodium hydroxide or potassium hydroxide is used to hydrolyze the ester. Typically, such hydrolysis is carried out in an aqueous medium, or a solvent system is employed that is a mixture of water and an appropriate alcohol. The product obtained from base-catalyzed hydrolysis is the salt form of the carboxylic acid group of cardiogenin. This salt can readily be converted to free acid, using a mineral acid.

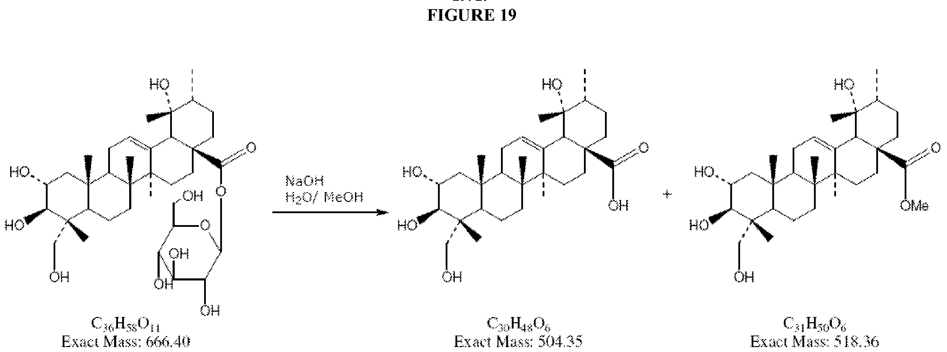

Figure 19 illustrates a conversion of the cardiogenin major isomer to the corresponding aglycone. The structure of the aglycone thus obtained is shown below:

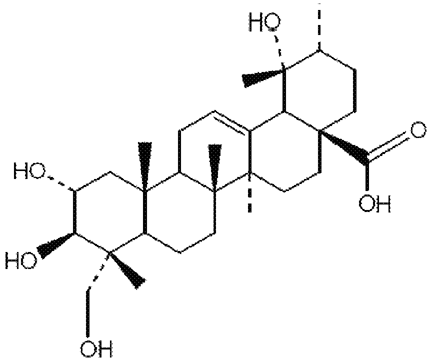

### INDUCING OR ENHANCING CARDIOGENIC DIFFERENTIATION

The cardiogenin major isomer and the corresponding aglycone can be used to induce or to enhance cardiogenic differentiation, both *in vitro* and *in vivo.* This utility is evidenced by the fact that MSCs cultured in the presence of a composition comprising the cardiogenin major isomer or its aglycone exhibit substantially enhanced differentiation into cardiomyocytes. In addition, beating cardiomyocytes differentiate from MSCs when the latter are co-cultured with cardiomyocytes in the presence of the cardiogenin major isomer or its aglycone.

### PHARMACEUTICAL COMPOSITIONS AND DOSAGES

The isolated cardiogenin major isomer and/or its corresponding aglycone can be administered, alone or with other compounds having similar or different biological activities. For instance, the compounds and pharmaceutical compositions described herein may be administered in a combination therapy, *i.e.,* either simultaneously in single or separate dosage forms or in separate dosage forms within hours or days of each other. Examples of such combination therapies include administering the compound of cardiogenin major isomer and/or its corresponding aglycone, with other agents used to treat stroke, skeletal muscle degeneration, wound healing, or cardiac problems.

Herein described is a pharmaceutical composition comprising the compound of cardiogenin major isomer, the hydrolytic free acid product thereof (*i.e.,* aglycone), or a pharmaceutically acceptable salt of the free acid, as well as a solvate, tautomer, polymorph, hydrate, structural derivative or prodrug thereof, in admixture with a pharmaceutically acceptable carrier. In some instances, the composition further contains, in accordance with accepted practices of pharmaceutical compounding, one or more additional therapeutic agents, pharmaceutically acceptable excipients, diluents, adjuvants, stabilizers, emulsifiers, preservatives, colorants, buffers, flavor imparting agents, absorption enhancers, complexing agents, solubilizing agents, wetting agents and surfactants.

In one instance, the pharmaceutical composition comprises a compound of cardiogenin major isomer or a pharmaceutically acceptable salt, solvates, tautomers, polymorphs, hydrates, structural derivative or prodrug thereof, and a pharmaceutically acceptable carrier.

In another instance, the pharmaceutical composition comprises an aglycone of cardiogenin major isomer or a pharmaceutically acceptable salt, solvates, tautomers, polymorphs, hydrates, structural derivative or prodrug thereof, and a pharmaceutically acceptable carrier.

The compositions can be administered orally, parenterally, by inhalation or spray, percutaneously, intravaginally, or rectally in dosage unit formulations. The term parenteral as used herein includes subcutaneous injections, intravenous, intramuscular, intrasternal, intrathecal, intraventricular, peritoneal, intracardiac injection or infusion techniques as well as via direct injection into any of numerous additional tissues or organs.

Compositions suitable for oral use may be prepared according to any method known to the art for the manufacture of pharmaceutical compositions. For instance, liquid formulations of the inventive compounds contain one or more agents selected from the group consisting of sweetening agents, solubilizers, dispersing agents, flavoring agents, coloring agents and preserving agents in order to provide pharmaceutically elegant and palatable preparations of the isomer.

For tablet compositions, the active ingredient in admixture with non-toxic pharmaceutically acceptable excipients is used for the manufacture of tablets. Examples of such excipients include without limitation inert diluents, such as calcium carbonate, sodium carbonate, lactose, carboxymethylcellulose, hydroxypropyl methylcellulose, mannitol, polyvinylpyrolidone, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known coating techniques to delay disintegration and absorption in the gastrointestinal tract and thereby to provide a sustained therapeutic action over a desired time period. For instance, a time-delay material such as glyceryl monostearate or glyceryl distearate may be employed. Additional tablet formulations that afford slow leaching of the active ingredient can be used to provide sustained release, including the use of hydrogels, osmotic pump tablets, and wax matrices.

Formulations for oral use may also be presented as hard or soft gelatin capsules wherein the active ingredient is mixed with an inert solid diluent, for example, calcium carbonate, calcium phosphate, lactose, mannitol, methycellulose or derivatives thereof, or kaolin, or as soft gelatin capsules wherein the active ingredient is mixed with water or an oil medium, for example peanut oil, liquid paraffin or olive oil.

For aqueous suspensions the present compound is admixed with excipients suitable for maintaining a stable suspension. Examples of such excipients include without limitation are sodium carboxymethylcellulose, methylcellulose, hydropropylmethylcellulose, sodium alginate, polyvinylpyrrolidone, gum tragacanth and gum acacia.

Oral suspensions can also contain dispersing or wetting agents, such as lecithin, or the condensation product of an alkylene oxide with fatty acids, for example polyoxyethylene stearate, or the product of ethylene oxide with long chain aliphatic alcohols, such as, heptadecaethyleneoxycetanol, or compounds such as polyoxyethylene sorbitol monooleate, or polyethylene sorbitan monooleate. The aqueous suspensions may also contain one or more preservatives, *e.g.,* ethyl or n-propyl p-hydroxybenzoate, as well as one or more coloring agents, one or more flavoring agents, and one or more sweetening agents, such as sucrose or saccharin.

Sweetening agents such as those set forth above, and flavoring agents may be added to provide palatable oral preparations. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, such as sweetening, flavoring and coloring agents, also may be present.

Pharmaceutical compositions may also be in the form of oil-in-water emulsions. The oily phase may be a vegetable oil, for example olive oil, sesame, peanut or arachis oil, or a mineral oil, for example liquid paraffin or mixtures of these. Suitable emulsifying agents include without limitation, naturally-occurring gums, for example gum acacia or gum tragacanth, other naturally-occurring compounds, for example, soy bean, lecithin, Tweens, and esters or partial esters derived from fatty acids and hexitol, anhydrides, sorbitan monoleate and polyoxyethylene sorbitan monoleate. The emulsions also may contain sweetening and flavoring agents.

Syrups and elixirs may be formulated with sweetening agents, for example glycerol, propylene glycol, sorbitol or sucrose. Such formulations may also contain a demulcent, a preservative, and flavoring and coloring agents. The pharmaceutical compositions may be in the form of a sterile injectable, an aqueous suspension or an oleaginous suspension. This suspension may be formulated according to the known art using those suitable dispersing or wetting agents and suspending agents which have been mentioned above. The sterile injectable preparation may also be sterile injectable solution or suspension in a non-toxic parentally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic monoglycerides or diglycerides. In addition, fatty acids such as oleic acid find use in the preparation of injectables.

### WORKING EXAMPLES - ISOLATION AND ACTIVITY OF CARDIOGENIN MAJOR ISOMER AND CORRESPONDING AGLYCONE

### 1. Extraction

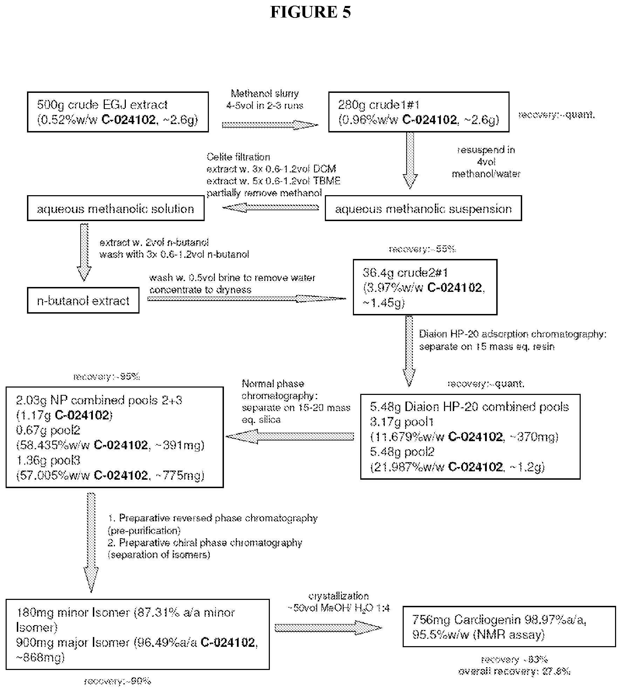

Figure 5 shows a schematic overview of the process of purifying the cardiogenin major isomer from EGJ. The whole plant, from which EGJ methanol extract is prepared, was collected from Guizhou Province of China. The plant also is known to be native to areas of Japan, Korea, and North America, and material collected from these areas is expected likewise to contain levels of cardiogenin that are suitable for sources of EGJ capable of affording high purity cardiogenin, via the purification process of the present description.

The collected material was dried and percolated with methanol at room temperature three times, for 6 days each time. The EGJ methanol extract was dried under reduced pressure using a spray drying procedure to yield a powder residue. 500g EGJ extract was stirred in 2.5L methanol at Ti = 41°C for 1h. After slow cooling to room temperature, the suspension was filtered off and rinsed with 200 ml methanol. The filter cake was resuspended in 1.5L methanol at Ti = 41°C for 1 h and stirred for an additional 20 hours at room temperature. After filtration, the filter cake was rinsed with 750 ml methanol. The combined methanol filtrates were concentrated to yield 280g crude #1.

Crude #1 was stirred in 1L of TBME plus 1.2L of water, resulting in a thick emulsion. The thick emulsion was diluted with 0.5L of methanol and filtered, the filter cake was rinsed intensively with 0.5L methanol and the combined filtrates (3.2L) were concentrated to a final volume of 2L. At this point a fine aqueous methanolic suspension had formed which could be filtered over a paper filter without pressure to furnish a clear dark, homogenous solution. The aqueous methanolic solution was extracted 3 times with 0.3L of dichloromethane. The aqueous methanolic solution was then extracted 5 times with 0.3L TBME portions. The remaining aqueous methanolic layer was diluted with 0.9L n-butanol, followed by 0.8L of water, to result in good phase separation.¹ The aqueous layer then was washed three times with 0.3L of n-butanol. The combined n-butanol layers (approximately 3.5L) were washed with 0.5L brine furnishing 1.4L of aqueous layer and 2.5L of n-butanol layer. The n-butanol was concentrated at the rotary evaporator removing approximately 0.5L of solvents. The remaining n-butanol layer (approximately 2L) was extracted twice with 0.3L of brine. Complete concentration of the n-butanol layer eventually furnished 36.4g of crude #2.
¹Alternatively, the aqueous methanolic suspension can be filtered with the aid of Celite with cake washed. The aqueous methanolic phase is next extracted 3 times with 0.3-0.6L of dichloromethane and 5 times with 0.3-0.6L TBME portions. The aqueous methanolic phase is rendered into a sodium chloride saturated solution and finally extracted with n-butanol. This process reduces emulsion formation during the DCM extraction and avoids need to concentrate aqueous methanolic phase in an attempt to remove methanol, avoiding severe foaming.

### 2. HP-20 Adsorption Chromatography

An amount of 545g dry HP-20 resin (approximately 15 mass equivalents dry resin per crude #2) was slurried in methanol, transferred to the column (7.5 cm × 21 cm = 927 ml column volume), and exchange for 20% methanol/water. Crude #2 was resuspended in approximately 1-1.5 vol. of 20% methanol/water and applied on the column. It was eluted with increasing concentration of methanol in water (10% step). For the 20-30% methanol/ water steps, 2 fractions of 2L were taken; for the 40-70% methanol/water steps, four 1L fractions were taken.

The fractions were analyzed by HPLC. Most cardiogenin content are in fraction 9-17. Fractions 9 and 10 were pooled together as pool-1; fractions 11-17 were pooled as pool-2; and fractions 1-8 and 18-20 were discarded. After concentration to dryness, pool-1 yielded 3.17g solids containing approximately 370 mg of cardiogenin, while pool-2 yielded 5.48g solids containing approximately 1.2g of cardiogenin.

### 3. Normal Phase Flash Chromatography

Pool-2 solids (5.4g) were slurried in 30 ml starting eluent DCM/methanol 90:10 to prepare the feed. Separation was performed on 100g silica gel (approximately 20 mass equivalents) equilibrated with DCM/methanol 90:10 (v:v). After allowing for 150 ml forerun, fractions were taken in 50 ml aliquots until fraction 32, then the eluent was changed for DC/methanol 85:15 and fractions of 100 ml size were taken. At fraction 42 the eluent was changed for DCM/ methanol 80:20.

Fractions 38 and 39 were pooled together as pool-11; fractions 40-44 were pooled as pool-22; and fractions 45-51 were pooled as pool-33. After concentration to dryness, pool-11 yielded 0.11g solids containing approximately 21 mg of cardiogenin (18.9%w/w); pool-22 yielded 0.67g solids containing approximately 391 mg of cardiogenin (58.4%w/w); and pool-33 yielded 1.36g solids containing approximately 775 mg of cardiogenin (57.0%w/w).

### 4. Reverse Phase Chromatography

To increase the overall purity of cardiogenin, reserve phase separation was performed using the following parameter:

| | |
|---|---|
| Column | 250x21.2 mm, 5 µm, Luna® C18(2) |
| Sample | 300 ul of 47 g/L in MeOH:Buffer=50:50 (v:v) |
| Eluent | A:ACN; B:20 mM NH₄OAc, pH=7 |
| Temperature | Room temperature |
| Flow rate | 15 mL/min |
| Detection | 210 nm |

The isolation procedure consisted of evaporating the ACN at the rotary evaporator at 40°C and reduced pressure and subsequently lyophilizing the remaining solution. The isolated foam was re-dissolved in ACN/water approximately 1:2 (v:v) and lyophilized again to remove residual traces of NH₄OAc. Pool-22 and pool-33 were combined and separated in 60 runs and yielded 1.29 g of white foam. As shown in Figure 4, the 1.29g mixture comprises two isomers having a combined HPLC purity of 92.6 % (a/a).

### 5. Chiral Phase Chromatography

To separate the two isomers in the 1.29g mixture, chiral phase separation was performed using the following parameter:

| | |
|---|---|
| Column | 192x25 mm, 20 µm, Chiralpak® IC™ |
| Sample | 3.5 mL of approximately 20 g/L in IPA |
| Eluent | isocratic; IPA:MTBE=50:50 (v:v) |
| Temperature | Room temperature |
| Flow rate | 25 mL/min |
| Detection | 210 nm |

Fractions 3 and 4 comprises primarily the major isomer of cardiogenin, while fraction 1 and 2 comprise primarily the minor isomer of cardiogenin. Fractions 2 and 3 were evaporated to dryness and re-processed with the same method to ensure a maximum yield. Fractions 1 and 2 and fractions 3 and 4 are combined, respectively, and worked up. The workup consisted again of evaporation to dryness followed by a lyophilization step after dissolution in water:ACN=75:25 (v:v). The workup gave 900 mg of white powder for the major isomer (96.49% a/a), and 180 mg of white powder for minor isomer.

### 6. Stress Tests

Stress tests were performed to determine whether the two cardiogenin isomers separated from each other are stable and not converting into each other. In particular, the two cardiogenin isomers were stressed by storage in MTBE/IPA=50:50 or as solids at 40°C (the major isomer in solution and solid; the minor isomer only in solution) for 24 hours.

All single impurity peaks of the cardiogenin major isomer and also the ratio of the two isomers were identical within the accuracy of the measurement. The same picture was obtained for the cardiogenin minor isomer. No changes were found in the composition within the accuracy of the method. It can be concluded that the two cardiogenin isomers are stable compounds, which resist thermal stress well.

### 7. Crystallization

Isolated cardiogenin major isomer as white powder (900 mg) was stirred in 10 volumes of methanol at To = 40 °C until a clear solution was obtained. Water (40 volumes) was added slowly, within 50 minutes, at To = 40 °C. After about 6 drops of water, crystallization started. After complete addition the thick white suspension was cooled to room temperature and filtered, and residual solids were flushed from the flask with small amounts of mother liquor. The filter cake was washed with methanol/ water (1:4) and dried at the rotary evaporator to furnish 756 mg cardiogenin as white solid crystal.

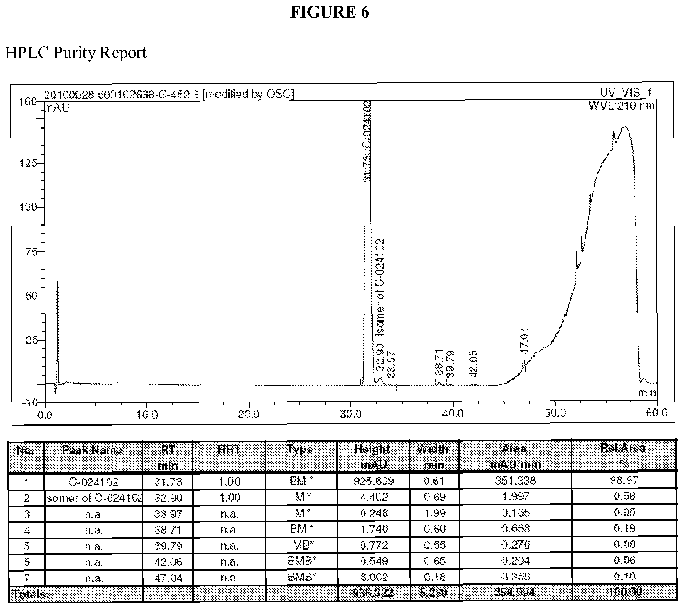

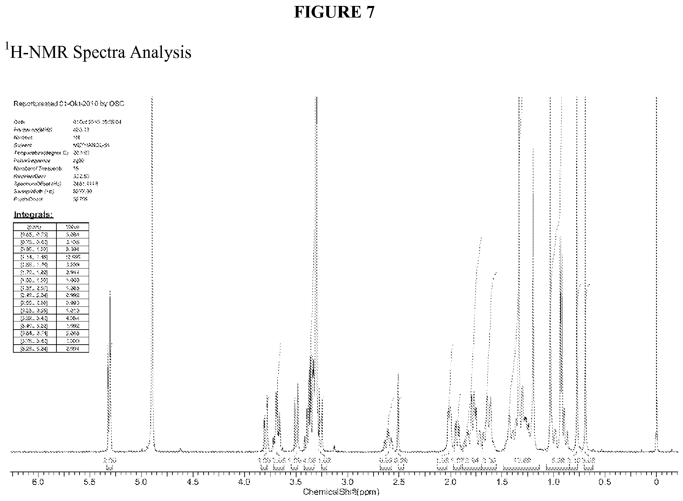

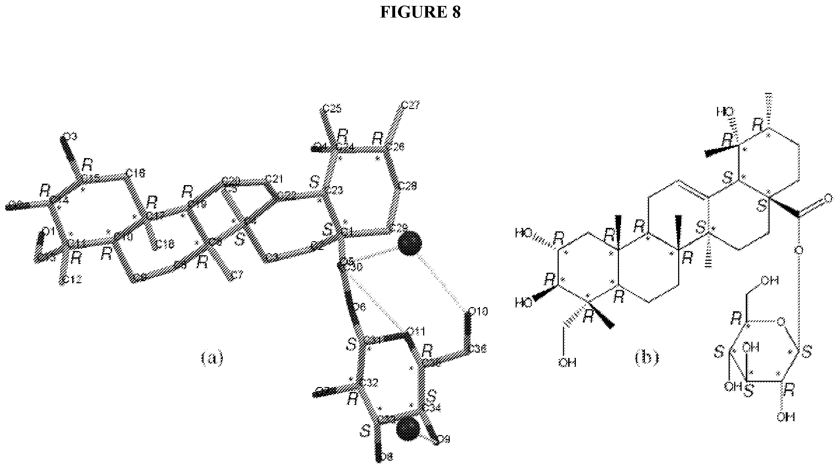

The purity of the isolated cardiogenin major isomer after crystallization is 98.97% a/a, as shown by HPLC at 210 nm (see Figure 6), and its identity is confirmed by ¹H-NMR spectra analysis (see Figure 7). The structure of the purified cardiogenin major isomer is confirmed by X-ray crystallography and illustrated in Figure 8. In addition, ¹H-NMR assay shows the potency of the purified cardiogenin major isomer to be 95.50% w/w.

### 8. Saponification

The isolated cardiogenin major isomer was subjected to saponification in MeOH:H₂O 1:1, using excess NaOH, as shown in Figure 19. The resultant aglycone of the isolated cardiogenin major isomer has a purity of at least 92% by HPLC.

### 9. Biological Activity

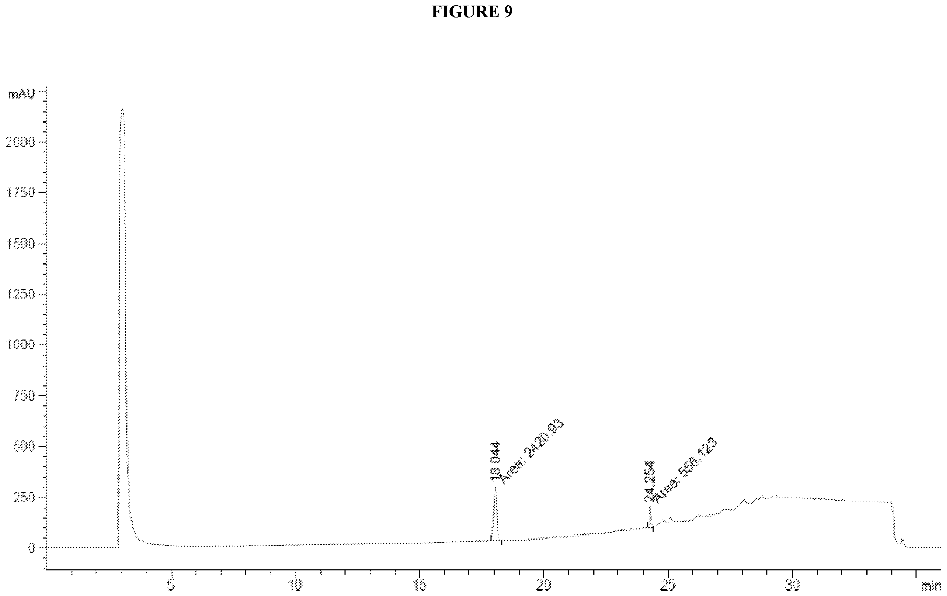

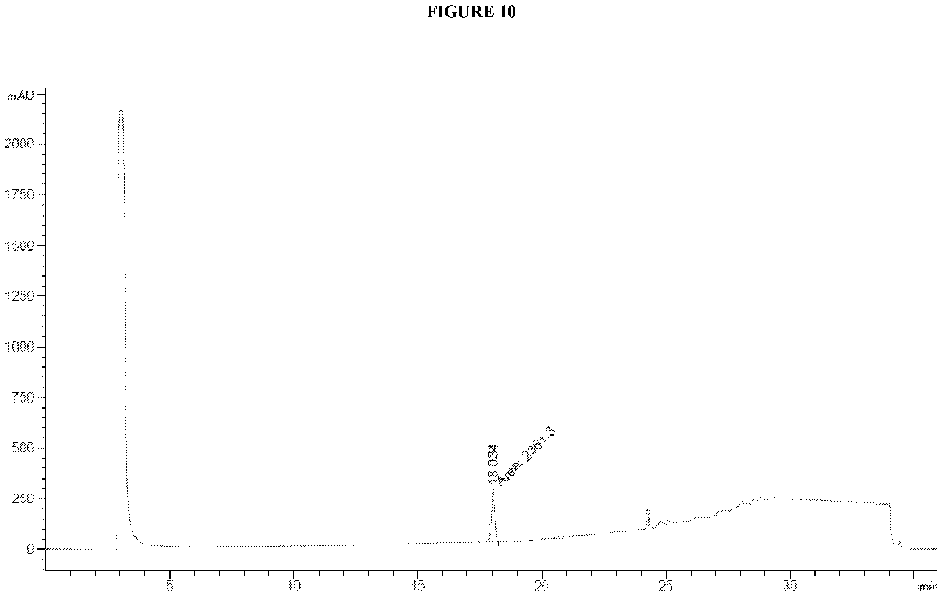

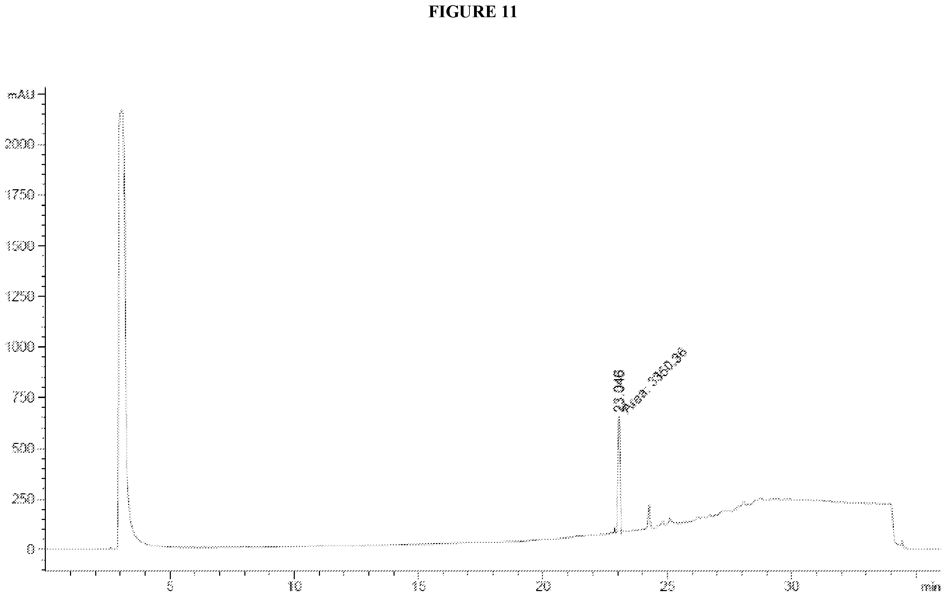

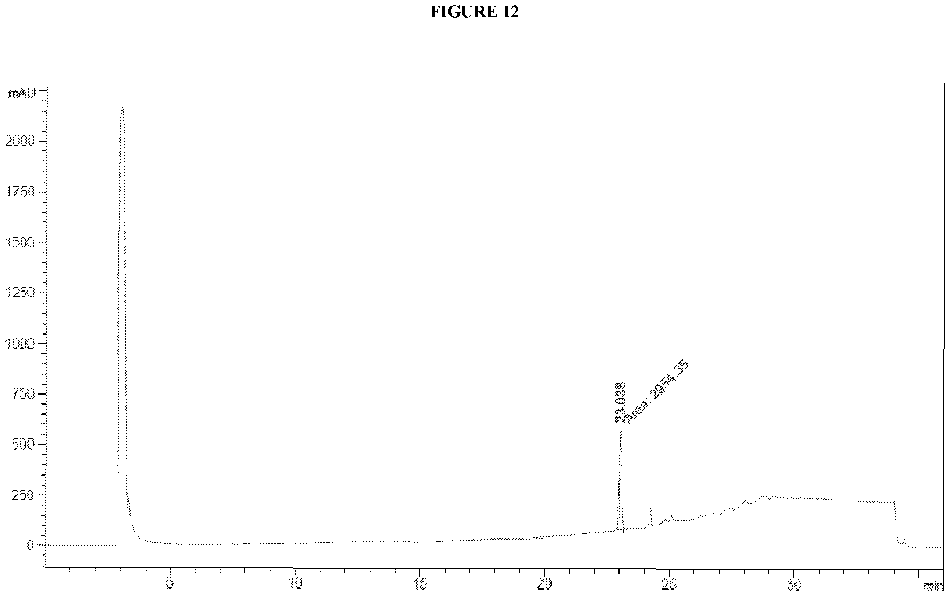

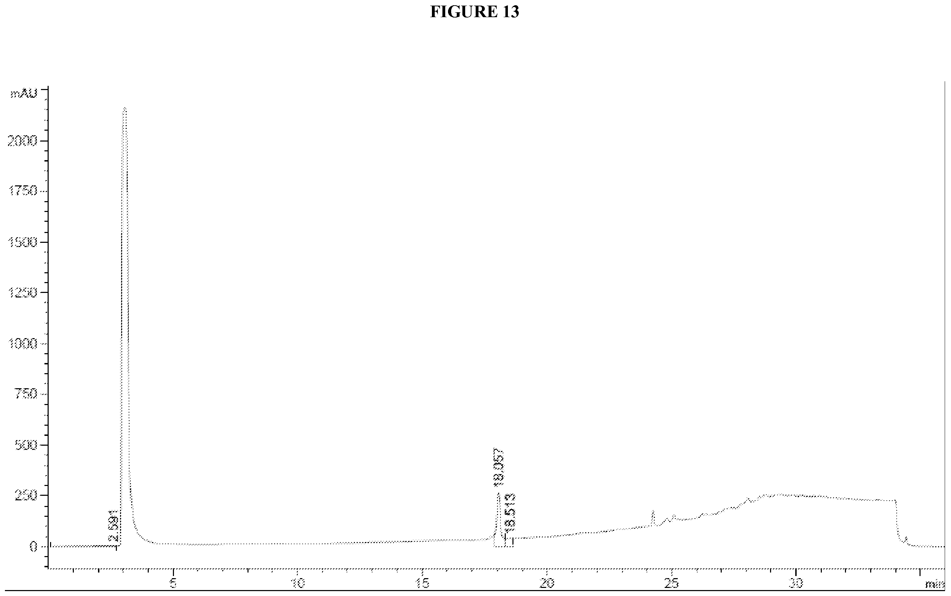

Tested for activity in inducing cardiogenic differentiation of MSCs were five compounds: the isolated cardiogenin major isomer (HUYA-1), the isolated cardiogenin minor isomer (HUYA-2), the aglycone of the cardiogenin minor isomer (HUYA-3), the aglycone of the cardiogenin major isomer (HUYA-4), and the cardiogenin composition prepared according to the method of Cheng *et al.,* 2009 (Car). In Figures 9 - 13, respectively, a C18 reverse phase chromatography profile is shown for these compounds.

Testing was performed according to the procedures described in the following: The tibias/femur bones of rats were removed and the BM was flushed out of the bones with alpha IMDM culture medium. The BM was mixed well and centrifuged at 1,500 rpm for 5 minutes. The cell pellet was suspended with 3 ml culture medium, and the forming cell suspension was carefully put on 4 ml Ficoll solution, to minimize disturbance, and then was centrifuged at 200 rpm for 30 minutes. The second layer was transferred into a tube and washed twice with PBS to remove Ficoll (1,200 rpm for 5 minutes). The resulting cell pellet was resuspended in IMDM culture medium containing 10% heat-inactivated FBS (GIBCO) and 1% penicillin/streptomycin antibiotic mixture, and this was used for the tests. Non-adherent cells were discarded after 24 hours culturing. The adherent cells were cultured by changing medium every 3 days. The cells became nearly confluent after 14 days culture. To activate the cardiogenic morphology transition, the MSCs were cultured for 7 days in the presence of the HUYA-1, HUYA-2, HUYA-3, HUYA-4 or Car (10 µg/ml IMDM culture medium), respectively. The treating period-dependent morphological transition was evaluated, on a time-lapse basis, with a phase contrast microscope.

After 3- or 7-day treatment of the MSCs in culture with HUYA-1, HUYA-2, HUYA-3, HUYA-4 or Car (10 µg/ml IMDM culture medium), respectively, fluorescent immunocytochemistry was performed, using antibodies specific to early cardiogenic differentiation factor 2 (MEF2a) at 3 days post-treatment and the contractile protein myosin heavy chain beta (MHC beta) at 7 days post-treatment, in order to demonstrate the cardiogenic differentiation of the treated MSCs in vitro. The method for the fluorescent-immunostaining is summarize briefly: The cultured cells were fixed with 4% paraformaldehyde in PBS for 15 minutes and permeabilized with 0.5% Triton X-100 for 15 minutes. Dilution of antibodies was as follows: rabbit polyoclonal antibodies specific to rat MEF2a (1:500) and mouse monoclonal antibodies specific to MHC (1:500) (both antibodies from abcam®). Secondary antibodies were goat anti-mouse and rabbit anti-IgG antibodies conjugated with fluorophore (FITC 495/528 and Cy5 650/667, products of abcam®), respectively. The nuclei were stained with DAPI. Examined by fluorescent microscopy were the cardiogenic differentiation-associated morphological transition and specific marker protein expression of the cultured MSCs.

Bone marrow GFP-MSCs were isolated from the tibias/femur bones of GFP-transgenic mice. The GFP-MSCs were co-cultured with the cardiac myocytes isolated from neonatal SD rats in the presence of HUYA-1 (10 µg/ml IMDM culture medium) to mimic the cardiac micro-environment. The cultures were investigated daily, under a fluorescent microscope, to identify beating GFP-positive cells and to gauge the morphological transition of the cultured GFP-MSCs.

As shown in Figures 14 and 15, isolated cardiogenin major isomer (HUYA-1) was the most active compound, inducing more than 20% of the cultured MSCs into cardiogenic differentiation in cell culture. HUYA-1 was more active than the cardiogenin composition made pursuant to Cheng *et al.,* 2009 (Car). The aglycone of the cardiogenin major isomer (HUYA-4) also induced MSCs into cardiogenic differentiation. Its lesser activity compared with HUYA-1 may be due to the lower solubility of HUYA-4 in cell culture medium. The DMSO concentration was increased from 5% to 10% to increase the solubility of HUYA-4. By comparison, isolated cardiogenin minor isomer (HUYA-2) and its aglycone (HUYA-3) were essentially inactive, with only *de minimus* induction observed of cardiogenic MSC differentiation.

Figure 18 demonstrates that HUYA-1 induced cardiogenic differentiation of the GFP-MSCs to form beating cardiac myocytes in the co-culture system.

## Claims

1. An improved method of extracting a compound from *Geum japonicum* having the formula 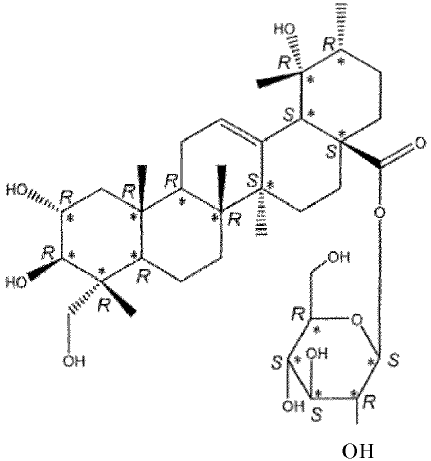 and having at least 98% (a/a) HPLC purity at 210 nm,
comprising the steps of (A) precipitating and filtering an methanolic/water solution of *Geum japonicum* to remove of unwanted solids, (B) phase separative extracting the methanol/water solution with dichloromethane and TBME, and (C) extracting with n-butanol.

2. The method according to claim 1 further comprising subjecting the extract of claim 1 to low pressure Diaion HP-20 adsorption chromatography using an optimal mass ration of resin to material load (15:1) and a step gradient of methanol/water, followed by low pressure silica gel chromatography using an optimal mass ration of resin to material load (20:1) and a step gradient of dichloromethane/methanol.

3. The method according to claim2 further comprising adding the step of high pressure reverse phase chromatography using aqueous buffer and methanol mobile phases in a gradient program.

4. The method according to claim 3, wherein the high pressure reverse phase chromatography comprises using a Luna® CI 8 (2) column.

5. A method of isolating the compound as defined in claim 1, comprising the steps of (A) obtaining an extract from the methanol extract of *Geum japonicum* and (B) subjecting the extract to chiral phase chromatography whereby the compound is obtained, and wherein the chiral phase chromatography comprises using a Chiralpak® IC™ column.

6. The method of claim 5, wherein an eluent for use with the chiral phase chromatography is a mixture of IPA:MTBE in a ratio of 50:50 (v:v).

7. The method of claim 5 or 6wherein the extract of step (A) is dissolved in a mixture of IPA:MTBA in a ratio of 50:50 (v:v) or neat IPA.

8. The method according to claim 5, further comprising (C) crystallizing the compound.

## Patentansprüche

1. Verbessertes Verfahren zum Extrahieren einer Verbindung aus *Geum japonicum* mit der Formel 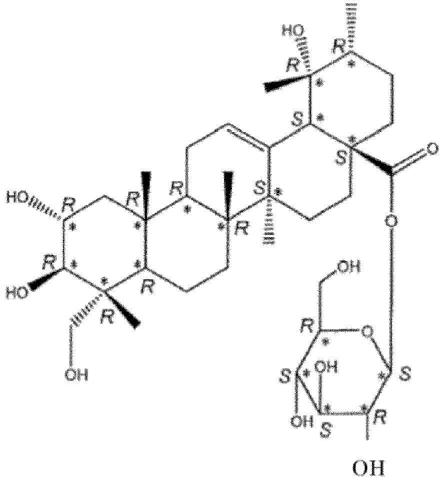 und mit einer HPLC-Reinheit von mindestens 98% (a/a) bei 210 nm,
umfassend die Schritte (A) Ausfällen und Filtern einer Methanol/Wasser-Lösung aus *Geum japonicum* zum Entfernen unerwünschter Feststoffe, (B) phasenseparatives Extrahieren der Methanol/Wasser-Lösung mit Dichlormethan und TBME, und (C) Extrahieren mit n-Butanol.

2. Verfahren nach Anspruch 1, ferner umfassend, den Extrakt nach Anspruch 1 einer Niederdruck-Adsorptionschromatographie mit Diaion HP-20 mithilfe eines optimalen Massenverhältnisses von Harz zu Materialbeschickung in Höhe von (15:1) und eines Stufengradienten von Methanol/Wasser, gefolgt von einer Niederdruck-Silicagel-Chromatographie mithilfe eines optimalen Massenverhältnisses von Harz zu Materialbeschickung in Höhe von (20:1) und eines Stufengradienten von Dichlormethan/Methanol.

3. Verfahren nach Anspruch 2, ferner umfassend das Hinzufügen des Schrittes einer Hochdruck-Rückwärtsphasenchromatographie unter Verwendung eines wässrigen Puffers und von Methanol-Mobilphasen in einem Gradientenprogramm.

4. Verfahren nach Anspruch 3, wobei die Hochdruck-Rückwärtsphasenchromatographie die Verwendung einer Säule Luna® CI 8 (2) umfasst.

5. Verfahren zur Isolierung der Verbindung nach Anspruch 1, umfassend die Schritte (A) Erhalten eines Extrakts ausgehend von dem Methanolextrakt aus *Geum japonicum* und (B) Unterziehen des Extrakts einer chiralen Phasenchromatographie, wodurch die Verbindung erhalten wird, wobei die chirale Phasenchromatographie die Verwendung einer Säule Chiralpak® IC™ umfasst.

6. Verfahren nach Anspruch 5, wobei es sich bei einem Elutionsmittel zur Verwendung mit der chiralen Phasenchromatographie um ein Gemisch aus IPA:MTBE in einem Verhältnis von 50:50 (v:v) handelt.

7. Verfahren nach Anspruch 5 oder 6, wobei der Extrakt aus Schritt (A) in einem Gemisch aus IPA:MTBA in einem Verhältnis von 50:50 (v:v) oder reinem IPA gelöst wird.

8. Verfahren nach Anspruch 5, ferner umfassend (C) das Kristallisieren der Verbindung.

## Revendications

1. Procédé amélioré d'extraction d'un composé issu de *Geum japonicum* présentant la formule 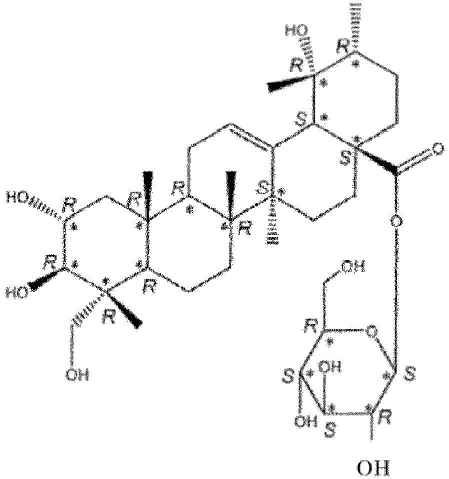 et présentant au moins 98 % (a/a) de pureté HPLC à 210 nm,
comprenant les étapes de (A) précipitation et filtrage d'une solution méthanol/eau de *Geum japonicum* pour enlever les solides non souhaités, (B) extraction par séparation de phases de la solution méthanol/eau avec du dichlorométhane et du TBME, et (C) extraction avec du n-butanol.

2. Le procédé selon la revendication 1, comprenant en outre la soumission de l'extrait de la revendication 1 à une chromatographie par adsorption au HP-20 Diaion à basse pression moyennant un rapport de masse résine - charge de matière optimal (15:1) et un gradient échelonné de méthanol/eau, suivie par une chromatographie au gel de silice à basse pression moyennant un rapport de masse résine - charge de matière optimal (20:1) et un gradient échelonné de dichlorométhane/méthanol.

3. Le procédé selon la revendication 2, comprenant en outre l'ajout de l'étape de chromatographie sur phase inverse à haute pression moyennant un tampon aqueux et des phases mobiles de méthanol dans un programme de gradient.

4. Le procédé selon la revendication 3, sachant que la chromatographie sur phase inverse à haute pression comprend l'utilisation d'une colonne Luna® CI 8 (2).

5. Procédé d'isolation du composé tel que défini dans la revendication 1, comprenant les étapes de (A) obtention d'un extrait à partir de l'extrait de méthanol de *Geum japonicum* et (B) soumission de l'extrait à une chromatographie sur phase chirale, de façon à obtenir ainsi le composé, et sachant que la chromatographie sur phase chirale comprend l'utilisation d'une colonne Chiralpak® IC™.

6. Le procédé selon la revendication 5, sachant qu'un éluent destiné à être utilisé avec la chromatographie sur phase chirale est un mélange de IPA:MTBE dans un rapport de 50:50 (v:v).

7. Le procédé selon la revendication 5 ou 6, sachant que l'extrait de l'étape (A) est dissout dans un mélange de IPA:MTBA dans un rapport de 50:50 (v:v) ou dans de l'IPA pur.

8. Le procédé selon la revendication 5, comprenant en outre (C) la cristallisation du composé.
